# EUROPEAN PATENT APPLICATION

(11) **EP 4 736 776 A1**
(43) Date of publication of application: **06.05.2026**
(21) Application number: 25211994.6
(22) Date of filing: 29.10.2025
(51) Int. Cl.: A61B 8/00, A61B 8/08

(54) **ULTRASOUND DIAGNOSIS SUPPORT APPARATUS AND ULTRASOUND DIAGNOSIS SUPPORT PROGRAM**

(30) Priority: 30.10.2024 JP 2024190802
(71) Applicant: FUJIFILM Corporation, Tokyo 106-8620 (JP)
(72) Inventor: SATO, Yuko, Tokyo (JP); WAKI, Koji, Tokyo (JP)
(74) Representative: Meissner Bolte Partnerschaft mbB

(57) **Abstract**

Provided is a technique that enables a user to easily understand a region in a target tissue of a subject that has been scanned with an ultrasound beam and a region that has not been scanned with the ultrasound beam.

A three-dimensional model forming unit acquires a three-dimensional model of a target tissue in which each position is represented by coordinates in a model coordinate system. A probe position and posture detection unit acquires a position and a posture of an ultrasound probe. A position and posture information conversion unit converts position and posture information indicating the position and the posture of the ultrasound probe into converted position and posture information in the model coordinate system. A scanned region specifying unit specifies a model scanning plane in the model coordinate system based on the converted position and posture information in a case in which the target tissue is scanned with an ultrasound beam, and specifies a region of the three-dimensional model through which the model scanning plane passes as a scanned region. A display controller displays the scanned region and an unscanned region in the three-dimensional model in different display modes.

## Description

### BACKGROUND OF THE INVENTION

### 1. Field of the Invention

The present specification discloses improvements to an ultrasound diagnosis support apparatus and an ultrasound diagnosis support program.

### 2. Description of the Related Art

In the related art, there is known an ultrasound diagnostic apparatus that forms an ultrasound tomographic image showing the inside of a subject based on reflected waves from the subject in a case in which ultrasonic waves are transmitted to the subject. Specifically, an ultrasound beam is emitted from an ultrasound probe to scan an ultrasound scanning plane, and an ultrasound tomographic image showing a cross section of the subject on the ultrasound scanning plane is formed based on the reflected waves of the ultrasound beam from the subject.

In the related art, various technologies related to ultrasound diagnostic apparatuses have been proposed.

For example, JP2021-53379A discloses an ultrasound diagnostic apparatus having a position sensor that detects a position of an ultrasound probe, in which the ultrasound diagnostic apparatus estimates an object region based on a plurality of ultrasound images obtained by transmitting and receiving ultrasound waves to and from an object within a subject and position information of the ultrasound probe detected by the position sensor, and further specifies an area of the object region where ultrasound images have not yet been captured based on the plurality of ultrasound images and the position information.

Furthermore, JP2021-29675A discloses an ultrasound diagnostic apparatus having a camera, in which the ultrasound diagnostic apparatus estimates a position and a posture of a subject from a first exterior image obtained by imaging the subject with the camera, determines a position and a posture of an ultrasound probe from a second exterior image obtained by imaging an AR marker attached to the ultrasound probe with the camera, and specifies an examination site based on the position and the posture of the subject and the position and the posture of the ultrasound probe.

### SUMMARY OF THE INVENTION

In a case in which a target tissue of a subject is scanned with an ultrasound beam, an operator such as a doctor, or a user or an administrator of the ultrasound image (collectively referred to as "user" in the present specification) may want to understand which portion of the target tissue has been scanned with the ultrasound beam, that is, which portion of the target tissue has already acquired a corresponding received signal.

Although it is merely an example, before performing a treatment such as removing a tumor on a target tissue, an operator may check an ultrasound tomographic image showing the tumor inside the target tissue to check a position or a state of the tumor. In this case, it is necessary to form an ultrasound tomographic image that appropriately shows the tumor by scanning an ultrasound scanning plane that includes the tumor with an ultrasound beam. In particular, in a case in which a plurality of tumors are scattered in the target tissue, it is preferable to appropriately form a plurality of ultrasound tomographic images showing each tumor. In such a case, it is desirable for the user to be able to understand whether the ultrasound scanning plane including the tumor has been appropriately scanned with the ultrasound beam such that an appropriate ultrasound tomographic image can be formed.

An object of an ultrasound diagnosis support apparatus disclosed in the present specification is to enable a user to easily understand a region in a target tissue of a subject that has been scanned with an ultrasound beam and a region that has not been scanned with the ultrasound beam.

An ultrasound diagnosis support apparatus disclosed in the present specification comprises a processor, in which the processor is configured to: acquire a three-dimensional model of a target tissue formed based on medical volume data acquired by a medical apparatus other than an ultrasound diagnostic apparatus, each position of the three-dimensional model being represented by a coordinate in a model coordinate system; acquire position and posture information indicating a position and a posture of an ultrasound probe that scans an ultrasound scanning plane including the target tissue with an ultrasonic wave; convert the position and posture information into converted position and posture information in the model coordinate system; specify a model scanning plane that is a virtual ultrasound scanning plane in the model coordinate system based on the converted position and posture information in a case in which the target tissue is scanned with an ultrasound beam, and specify a region of the three-dimensional model through which the model scanning plane passes as a scanned region; and display the three-dimensional model on a display unit, and display the scanned region and an unscanned region, which is a region that has not been scanned with the ultrasound beam, in the three-dimensional model in different display modes.

Information indicating a position of a portion of interest in the target tissue may be added to the three-dimensional model, and the processor may be configured to display the information indicating the position of the portion of interest on the display unit.

The processor may be configured to: evaluate a received signal obtained by scanning an ultrasound scanning plane corresponding to the model scanning plane that defines the scanned region with the ultrasound beam; and display the scanned region in a manner in which an evaluation result of the received signal is known.

The processor may be configured to display, on a cross section as the scanned region defined by the model scanning plane, an ultrasound tomographic image formed based on a received signal obtained by scanning an ultrasound scanning plane corresponding to the model scanning plane with the ultrasound beam.

The processor may be configured to: display, on the display unit, a medical tomographic image reconstructed by cutting out the medical volume data at a cross section based on the converted position and posture information, and an ultrasound tomographic image formed based on a received signal acquired at a current position and posture of the ultrasound probe; and display a guide image indicating a range of the ultrasound scanning plane in a superimposed manner on the medical tomographic image.

The processor may be configured to detect a position and a posture of the ultrasound probe relative to a position and a posture of the target tissue based on a captured image obtained by imaging detection marks attached to the ultrasound probe and the target tissue with a camera.

In addition, an ultrasound diagnosis support program disclosed in the present specification causes a computer to: acquire a three-dimensional model of a target tissue formed based on medical volume data acquired by a medical apparatus other than an ultrasound diagnostic apparatus, each position of the three-dimensional model being represented by a coordinate in a model coordinate system; acquire position and posture information indicating a position and a posture of an ultrasound probe that scans an ultrasound scanning plane including the target tissue with an ultrasonic wave; convert the position and posture information into converted position and posture information in the model coordinate system; specify an ultrasound scanning plane in the model coordinate system based on the converted position and posture information in a case in which the target tissue is scanned with an ultrasound beam, and specify a region of the three-dimensional model through which the ultrasound scanning plane passes as a scanned region; and display the three-dimensional model on a display unit, and display the scanned region and an unscanned region, which is a region that has not been scanned with the ultrasound beam, in the three-dimensional model in different display modes.

According to the ultrasound diagnosis support apparatus disclosed in the present specification, a user can easily understand a region in a target tissue of a subject that has been scanned with an ultrasound beam and a region that has not been scanned with the ultrasound beam.

### BRIEF DESCRIPTION OF THE DRAWINGS

FIG. 1 is a schematic diagram showing a configuration of an ultrasound diagnosis support system according to the present embodiment.
FIG. 2 is a conceptual diagram showing a camera, an ultrasound probe, and a target tissue.
FIG. 3 is a schematic diagram showing a configuration of an ultrasound diagnostic apparatus according to the present embodiment.
FIG. 4 is a diagram showing an example of a three-dimensional model of a target tissue.
FIG. 5 is a diagram showing an example of a captured image obtained from a camera.
FIG. 6 is a diagram showing a display example of a medical tomographic image and an ultrasound tomographic image.
FIG. 7 is a diagram showing a first example of an ultrasound scanning plane in a model coordinate system.
FIG. 8 is a diagram showing a first example of a scanned region.
FIG. 9 is a diagram showing a second example of the ultrasound scanning plane in the model coordinate system.
FIG. 10 is a diagram showing a second example of the scanned region.
FIG. 11 is a diagram showing a third example of the scanned region.
FIG. 12 is a diagram showing a first display example of a three-dimensional model.
FIG. 13 is a diagram showing a second display example of the three-dimensional model.
FIG. 14 is a diagram showing a third display example of the three-dimensional model.
FIG. 15 is a diagram showing a display example of a three-dimensional model, a medical tomographic image, and an ultrasound tomographic image.

### DESCRIPTION OF THE PREFERRED EMBODIMENTS

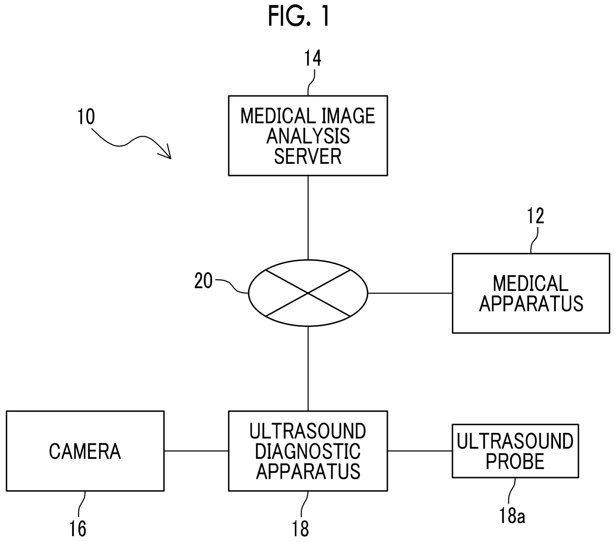

FIG. 1 is a schematic diagram showing a configuration of an ultrasound diagnosis support system 10 according to the present embodiment. The ultrasound diagnosis support system 10 includes a medical apparatus 12, a medical image analysis server 14, a camera 16, and an ultrasound diagnostic apparatus 18 as an ultrasound diagnosis support apparatus including an ultrasound probe 18a. The medical apparatus 12, the medical image analysis server 14, and the ultrasound diagnostic apparatus 18 are connected to each other via a communication line 20, such as a wide area network (WAN) or a local area network (LAN), to be communicable with each other. Further, the camera 16 and the ultrasound diagnostic apparatus 18 are connected to each other in a communicable manner in a wired or wireless manner.

The medical apparatus 12 is configured by, for example, a computed tomography (CT) apparatus or a magnetic resonance imaging (MRI) apparatus. In the present embodiment, the medical apparatus 12 is a device other than an ultrasound diagnostic apparatus. The medical apparatus 12 is a device that forms medical volume data about a target tissue of a subject. The target tissue is a tissue that is scanned with an ultrasound beam by the ultrasound diagnostic apparatus 18. In the present embodiment, after the target tissue is scanned with an ultrasound beam, the operator performs a treatment on the target tissue, and therefore it may also be said that the target tissue is a tissue that is treated by the operator. The medical volume data is data in which voxels, each having data, are arranged three-dimensionally. The medical volume data has position information for each voxel. In the present embodiment, the position of each voxel that constitutes the medical volume data is expressed by coordinates in a medical data coordinate system. Before the ultrasound diagnostic apparatus 18 scans the target tissue with an ultrasound beam, the medical apparatus 12 forms medical volume data including the target tissue and transmits the formed medical volume data to the medical image analysis server 14.

The medical image analysis server 14 is configured by a server computer comprising, for example, a processor, a memory, a communication interface, and the like. The medical image analysis server 14 is a device that analyzes various types of medical image data formed by various modalities, such as medical volume data formed by the medical apparatus 12, or ultrasound tomographic images or ultrasound volume data formed by the ultrasound diagnostic apparatus 18. The medical image analysis server 14 transmits medical volume data including the target tissue to the ultrasound diagnostic apparatus 18 before the ultrasound diagnostic apparatus 18 scans the target tissue with an ultrasound beam. The medical volume data may be transmitted directly from the medical apparatus 12 to the ultrasound diagnostic apparatus 18.

The camera 16 includes a processor, a communication interface, and the like, in addition to a lens or an image sensor. The camera 16 images the ultrasound probe 18a, particularly a probe detection mark (described in detail below) attached to the ultrasound probe 18a. A captured image is formed by the image sensor of the camera 16, and the captured image is transmitted in real time to the ultrasound diagnostic apparatus 18 via the communication interface of the camera 16.

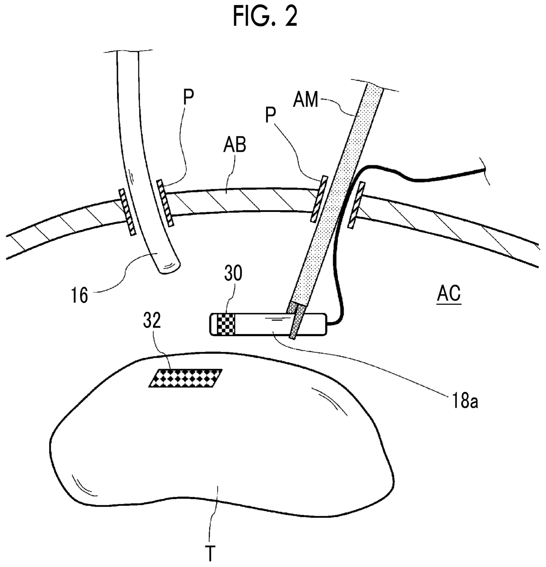

FIG. 2 is a conceptual diagram showing the camera 16, the ultrasound probe 18a, and a target tissue T of the subject (an organ in the example of FIG. 2). In the present embodiment, it is assumed that laparoscopic surgery is performed on the target tissue T. FIG. 2 shows a state in which the ultrasound probe 18a has been inserted into an abdominal cavity AC prior to laparoscopic surgery. A small hole is made in an abdomen AB of the subject, and a tube (port) P is attached to the hole. The abdominal cavity AC is inflated with gas, and the camera 16 and the ultrasound probe 18a are inserted into the abdominal cavity AC from the tube P. In the present embodiment, the ultrasound probe 18a is a drop-in type probe that is inserted into a body cavity (in the abdominal cavity AC in the example of FIG. 2). The ultrasound probe 18a scans an ultrasound scanning plane including the target tissue T with an ultrasound beam, thereby forming an ultrasound tomographic image of the target tissue T. In the present embodiment, the position of the camera 16 is fixed. The ultrasound probe 18a is gripped by an arm AM such as forceps held by an operator or a robot arm that operates under the control of the operator. That is, the position and the posture of the ultrasound probe 18a (that is, the ultrasound scanning plane) are controlled by the operator.

A probe detection mark 30 is attached to the ultrasound probe 18a. The probe detection mark 30 is a mark for detecting the position and the posture of the ultrasound probe 18a. The probe detection mark 30 is imaged by the camera 16 to acquire a captured image, and the position and the posture of the ultrasound probe 18a can be detected by analyzing the image of the probe detection mark 30 captured in the captured image. Examples of the probe detection mark 30 include an augmented reality (AR) marker.

In addition, an object detection mark 32 is attached to the surface of the target tissue T. The object detection mark 32 is a mark for detecting the position and the posture (on the surface) of the target tissue T. The object detection mark 32 is imaged by the camera 16 to acquire a captured image, and the position and the posture (on the surface) of the target tissue T can be detected by analyzing the image of the object detection mark 32 captured in the captured image. Examples of the object detection mark 32 include an AR marker. The object detection mark 32 has a pattern different from a pattern of the probe detection mark 30.

In the present embodiment, prior to treatment of the target tissue T (for example, removal of a tumor within the target tissue T), ultrasonic waves are transmitted and received to the target tissue T, and an ultrasound tomographic image or ultrasound volume data representing the target tissue T is formed based on the received signals obtained thereby. At this time, it is preferable that the user is able to understand a region in the target tissue T that has been scanned with the ultrasound beam and a region that has not been scanned with the ultrasound beam. For example, in a case in which there is a tumor in the target tissue T and the tumor is to be removed, the operator needs to scan the tumor with an ultrasound beam in order to check the state of the tumor using an ultrasound tomographic image before surgery (in other words, the tumor needs to be within the scanned region). In particular, in a case in which a plurality of tumors are scattered in the target tissue T, it is advisable to scan all of the plurality of tumors with the ultrasound beam from the viewpoint of preventing missed removal. In the present embodiment, the user is supported to easily understand the region of the target tissue T that has been scanned with the ultrasound beam and the region that has not been scanned with the ultrasound beam.

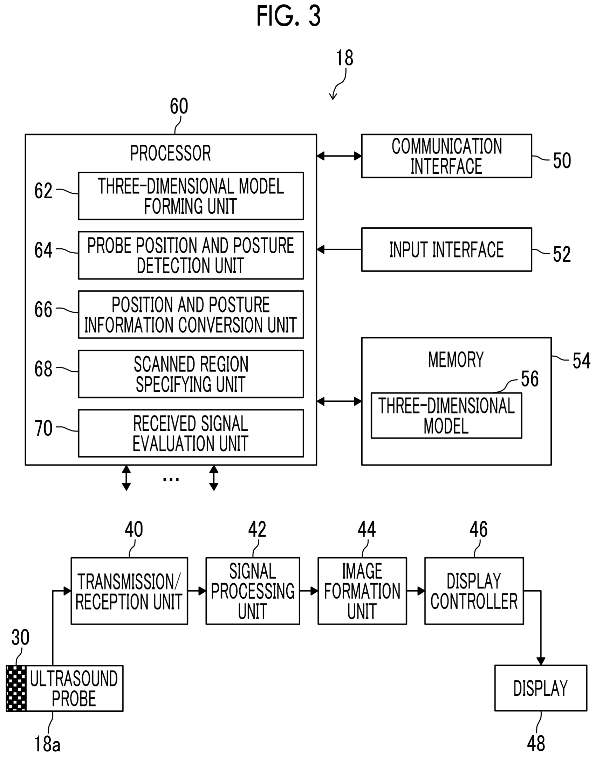

FIG. 3 is a schematic diagram showing the configuration of the ultrasound diagnostic apparatus 18. The ultrasound diagnostic apparatus 18 is a medical apparatus installed in medical institutions, such as a hospital.

The ultrasound probe 18a is a device that transmits and receives ultrasonic waves to and from the target tissue T of the subject. The ultrasound probe 18a has a transducer element array consisting of a plurality of transducer elements that scan the target tissue T with an ultrasound beam. In the ultrasound probe 18a, the transducer element array is formed of the plurality of transducer elements arranged in one row. In a case in which a transmission signal is supplied to each transducer element from a transmission/reception unit 40, which will be described later, each transducer element generates an ultrasound beam that scans an ultrasound scanning plane.

The size of the ultrasound scanning plane (lateral width, which is the length in the scanning direction of the ultrasound beam, and depth) is determined according to known information such as the structure of the ultrasound probe 18a and the settings of the ultrasound diagnostic apparatus 18. For example, the lateral width of the ultrasound scanning plane (scan width) is determined according to the number of transducer elements included in the ultrasound probe 18a. The depth of the ultrasound scanning plane (penetration depth) is determined according to the transmission strength of the ultrasound beam (that is, the strength of the transmission signal provided from the transmission/reception unit 40 to the ultrasound probe 18a) and other factors. The position and the posture (orientation) of the ultrasound scanning plane are determined based on the position and the posture of the ultrasound probe 18a. That is, since the size of the ultrasound scanning plane is determined by known information held by the ultrasound diagnostic apparatus 18, the ultrasound scanning plane can be defined in a case in which the position and the posture of the ultrasound probe 18a are known.

As described above, the probe detection mark 30 is attached to the ultrasound probe 18a.

The transmission/reception unit 40 transmits a transmission signal to the ultrasound probe 18a (specifically, to each transducer element of the transducer element array) under the control of a processor 60, which will be described later. In addition, the transmission/reception unit 40 receives a received signal from each transducer element that has received reflected waves from the target tissue T. The transmission/reception unit 40 includes an adder and a plurality of delay devices corresponding to each transducer element and performs phase alignment and addition processing of aligning and adding phases of the received signals from the transducer elements by using the adder and the plurality of delay devices. Accordingly, a reception beam signal is formed in which information indicating the signal intensity of the reflected waves from the target tissue T is aligned in the depth direction of the target tissue T.

A signal processing unit 42 executes various types of signal processing including filter processing of applying a bandpass filter, detection processing, and the like, on the reception beam signal from the transmission/reception unit 40.

An image forming unit 44 forms the ultrasound tomographic image (B-mode image) representing the cross section (particularly, an ultrasonic wave transmitting and receiving surface) of the target tissue T based on the reception beam signal on which the signal processing has been performed by the signal processing unit 42. Furthermore, the image forming unit 44 forms a medical tomographic image showing a cross section of the medical volume data based on the medical volume data received from the medical apparatus 12 or the medical image analysis server 14.

A display controller 46 performs control to display various images including the ultrasound tomographic image formed by the image forming unit 44 on a display 48.

The display 48 as a display unit is, for example, a display device configured using a liquid-crystal display, an organic electro luminescence (EL), or the like.

The transmission/reception unit 40, the signal processing unit 42, the image forming unit 44, and the display controller 46 of the ultrasound diagnostic apparatus 18 are configured by a processor (which may be the processor 60 described below or a processor different from the processor 60).

A communication interface 50 is configured by, for example, a network adapter. The communication interface 50 exhibits the function of communicating with other devices (particularly the medical apparatus 12, the medical image analysis server 14, and the camera 16) via the communication line 20. In particular, the communication interface 50 receives medical volume data from the medical apparatus 12 or the medical image analysis server 14 and receives a captured image from the camera 16.

An input interface 52 is configured by, for example, a button, a trackball, or a touch panel. The input interface 52 is used to input an instruction of an operator who uses the ultrasound diagnostic apparatus 18 to the ultrasound diagnostic apparatus 18.

A memory 54 includes a hard disk drive (HDD), a solid-state drive (SSD), an embedded multi media card (eMMC), a read-only memory (ROM), a random-access memory (RAM), or the like. An ultrasound diagnosis support program is stored in the memory 54 in order to operate each unit of the ultrasound diagnostic apparatus 18. In addition, the ultrasound diagnosis support program can also be stored in, for example, a computer-readable non-transitory storage medium such as a universal serial bus (USB) memory or a CD-ROM. The ultrasound diagnostic apparatus 18 can read the ultrasound diagnosis support program from such a storage medium and execute the ultrasound diagnosis support program.

Also, as shown in FIG. 3, the memory 54 stores a three-dimensional model 56. The three-dimensional model 56 is formed by a three-dimensional model forming unit 62 to be described later. Details of the three-dimensional model 56 will be described later.

The processor 60 exhibits the functions of a three-dimensional model forming unit 62, a probe position and posture detection unit 64, a position and posture information conversion unit 66, a scanned region specifying unit 68, and a received signal evaluation unit 70 in accordance with the ultrasound diagnosis support program stored in the memory 54. The functions of each unit exhibited by the processor 60 will be described in detail below.

The three-dimensional model forming unit 62 forms the three-dimensional model 56 of the target tissue T based on the medical volume data received from the medical apparatus 12 or the medical image analysis server 14. Accordingly, the three-dimensional model forming unit 62 acquires the three-dimensional model 56 of the target tissue T.

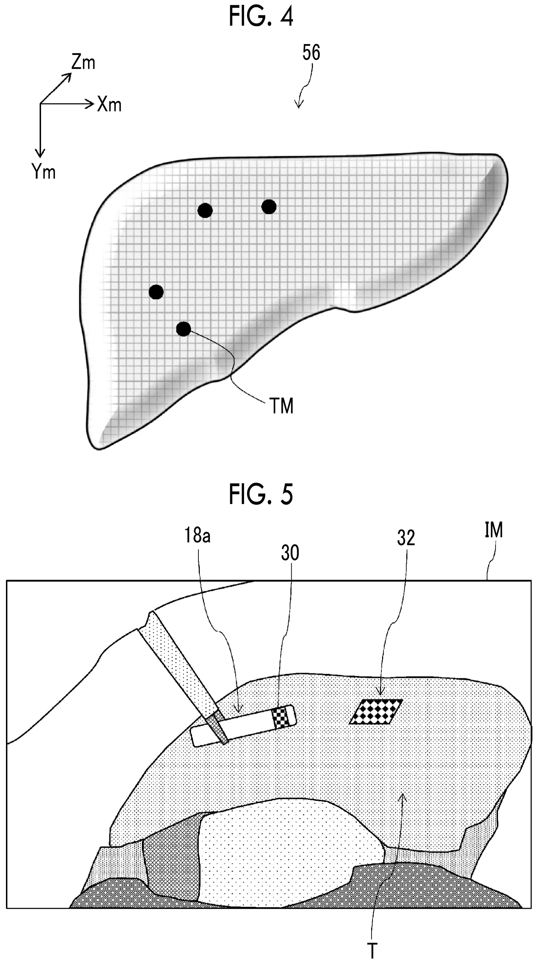

FIG. 4 is a diagram showing an example of the three-dimensional model 56 of the target tissue T. Hereinafter, the description will be provided assuming that the target tissue T is the liver. The three-dimensional model 56 is formed from medical volume data including the liver as the target tissue T. Since a known method can be used as a method of forming the three-dimensional model 56 based on the medical volume data, a detailed description will be omitted here, but the three-dimensional model forming unit 62 forms the three-dimensional model 56 using techniques such as volume rendering or surface rendering.

Since the medical volume data has the position information of each voxel, and the three-dimensional model 56 is formed from the medical volume data, the three-dimensional model 56 also has the position information (coordinates) indicating each position. Each position of the three-dimensional model 56 is represented by a coordinate in the model coordinate system (which is the same coordinate system as the medical data coordinate system). In each drawing in the present specification, a model coordinate system is represented by an Xm axis, a Ym axis, and a Zm axis.

Furthermore, information indicating the position of a portion of the target tissue T that the user should pay attention to, that is, a portion of interest, is added to the three-dimensional model 56. In the present embodiment, information indicating the position of a tumor TM within the target tissue T as a portion of interest is added to the three-dimensional model 56. The information indicating the position of the tumor TM may be set by automatic calculation in a case in which the three-dimensional model forming unit 62 forms the three-dimensional model 56, or may be set manually by an operator or the like. Since the tumor TM is not usually represented by the coordinates of a single point, the three-dimensional model 56 has information indicating a coordinate region occupied by the tumor TM (which may also be said to be a position and a size of the tumor). As shown in FIG. 4, in a case in which a plurality of tumors TM are scattered in the target tissue T, the three-dimensional model 56 has information indicating a plurality of coordinate regions corresponding to each tumor TM.

In addition, the three-dimensional model 56 may be formed by the medical apparatus 12 or the medical image analysis server 14, and the ultrasound diagnostic apparatus 18 may receive the formed three-dimensional model 56 from the medical apparatus 12 or the medical image analysis server 14. In this case, the processor 60 only needs to receive (acquire) the three-dimensional model 56 and does not need to exhibit the function of the three-dimensional model forming unit 62.

The probe position and posture detection unit 64 detects and acquires a position and a posture of the ultrasound probe 18a. In the present embodiment, the probe position and posture detection unit 64 detects the position and the posture of the ultrasound probe 18a based on the captured image formed by the camera 16. The position and the posture of the ultrasound probe 18a may constantly fluctuate due to the operation of the operator, but the probe position and posture detection unit 64 continuously detects the position and the posture of the ultrasound probe 18a.

FIG. 5 is a diagram showing an example of a captured image IM obtained from the camera 16. The captured image IM includes an image of the probe detection mark 30 that indicates the current position and posture of the ultrasound probe 18a. The probe position and posture detection unit 64 detects the current position and posture of the ultrasound probe 18a in the camera coordinate system of the camera 16 by analyzing the image of the probe detection mark 30 in the captured image IM. The camera coordinate system is a coordinate system with the position of the camera 16 as its origin, and is defined by three axes, for example, a z-axis being the optical axis direction of the lens of the camera 16, an x-axis being the direction perpendicular to the z-axis, and a y-axis being the direction perpendicular to the x-axis and the z-axis. Note that since a known method can be used as a method of detecting the position and the posture of the ultrasound probe 18a in the camera coordinate system from the image of the probe detection mark 30 included in the captured image IM, a detailed description will be omitted here. The position of the ultrasound probe 18a may be expressed, for example, by the coordinates of a representative point of the ultrasound probe 18a (for example, a position where the probe detection mark 30 is attached), and the posture of the ultrasound probe 18a may be expressed, for example, by the rotation angle around each of the three axis directions of the camera coordinate system.

The probe position and posture detection unit 64 may further detect the current position and posture of the target tissue T.

The captured image IM also includes an image of the object detection mark 32 that indicates the position and the posture of the target tissue T. The probe position and posture detection unit 64 detects the position and the posture of the target tissue T in the camera coordinate system of the camera 16 by analyzing the image of the object detection mark 32 in the captured image IM. Note that since a known method can be used as a method of detecting the position and the posture of the target tissue T in the camera coordinate system from the image of the object detection mark 32 included in the captured image IM, a detailed description will be omitted here. The position of the target tissue T may be expressed, for example, by the coordinates of a representative point of the target tissue T (for example, a position where the object detection mark 32 is attached), and the posture of the target tissue T may be expressed, for example, by the rotation angle around each of the three axis directions of the camera coordinate system.

Then, the probe position and posture detection unit 64 may detect the position and the posture of the ultrasound probe 18a relative to the position and the posture of the target tissue T (surface). This makes it possible to obtain the position and the posture of the ultrasound probe 18a relative to the target tissue T, absorbing any fluctuations in the position or the posture of the target tissue T.

The probe position and posture detection unit 64 sequentially detects the current position and posture of the ultrasound probe 18a based on the captured images IM (which may be frames constituting a moving image) sequentially sent in real time from the camera 16. In other words, it may also be said that the probe position and posture detection unit 64 detects changes in the position and the posture of the ultrasound probe 18a.

The position and the posture of the ultrasound probe 18a may be detected by a method other than analyzing the captured image IM. For example, the ultrasound probe 18a may be provided with a magnetic sensor or an acceleration sensor, and the processor 60 may acquire position and posture information indicating the current position and posture of the ultrasound probe 18a from these sensors. In this case, the position and the posture of the ultrasound probe 18a are expressed by coordinates in a real space coordinate system with an origin determined by sensor calibration as a reference.

The position and posture information conversion unit 66 converts the position and posture information indicating the position and the posture of the ultrasound probe 18a defined in the camera coordinate system or the real space coordinate system into position and posture information in the model coordinate system. In the present specification, the position and posture information of the ultrasound probe 18a converted into the model coordinate system is referred to as "converted position and posture information". An example of a method of converting into converted position and posture information is as follows.

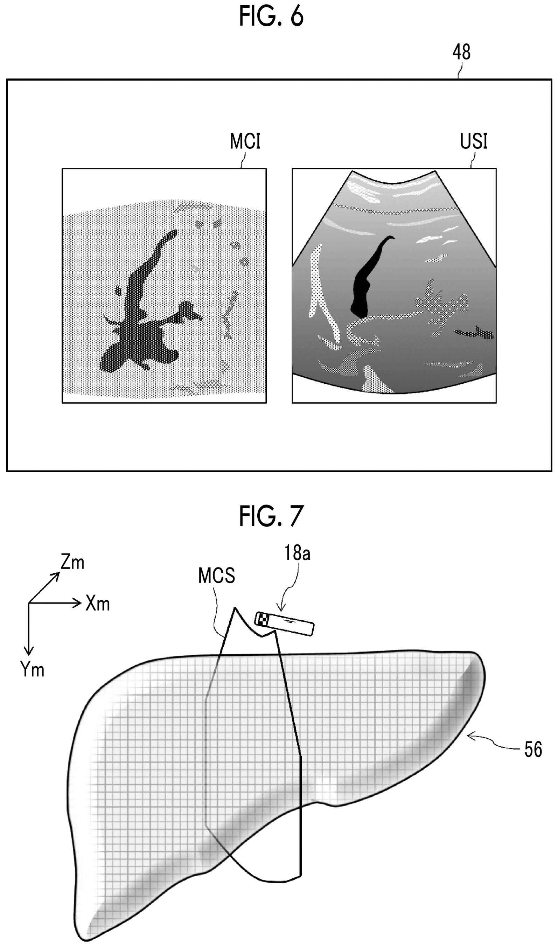

FIG. 6 is a diagram showing a display example of a medical tomographic image MCI and an ultrasound tomographic image USI. First, the image forming unit 44 forms the medical tomographic image MCI of a predetermined cross section of the medical volume data received from the medical apparatus 12 or the medical image analysis server 14. The predetermined cross section may be a cross section that shows a characteristic structure in the medical volume data and is automatically selected by the image forming unit 44, or may be a cross section that is designated by the operator.

In addition, the image forming unit 44 forms an ultrasound tomographic image (that is, a real-time ultrasound tomographic image) USI representing a cross section of the target tissue T on the ultrasound scanning plane based on the received signal obtained as the ultrasound probe 18a in its current position and posture scans the target tissue T with an ultrasound beam on the ultrasound scanning plane.

The display controller 46 then causes the display 48 to display the medical tomographic image MCI and the ultrasound tomographic image USI thus formed. In order for the operator to easily compare the two images, the display controller 46 may display the medical tomographic image MCI and the ultrasound tomographic image USI side by side. Since the ultrasound tomographic image USI is a real-time ultrasound tomographic image, the ultrasound scanning plane changes in a case in which the position and the posture of the ultrasound probe 18a change, and thus the content of the ultrasound tomographic image USI changes dynamically depending on the position or the posture of the ultrasound probe 18a.

Here, the operator compares the medical tomographic image MCI and the ultrasound tomographic image USI displayed on the display 48, and adjusts the position and the posture of the ultrasound probe 18a such that the cross section of the ultrasound tomographic image USI is the same as the cross section of the medical tomographic image MCI. In a case in which the cross section of the ultrasound tomographic image USI is the same as the cross section of the medical tomographic image MCI, the predetermined cross section corresponding to the medical tomographic image MCI designated in the medical volume data and the ultrasonic wave transmitting and receiving surface are the same cross section. Here, the predetermined cross section corresponding to the medical tomographic image MCI is designated in the medical volume data, and therefore can be expressed in the medical data coordinate system, that is, the model coordinate system. On the other hand, the position and the posture of the ultrasound probe 18a are expressed in a camera coordinate system or a real space coordinate system, and the ultrasonic wave transmitting and receiving surface is determined based on the position and the posture of the ultrasound probe 18a. Therefore, in a case in which the cross section of the ultrasound tomographic image USI is the same as the cross section of the medical tomographic image MCI, the operator can input a calibration instruction to the processor 60, and a relationship between the model coordinate system and the camera coordinate system or the real space coordinate system can be acquired from the position and posture relationship between a predetermined cross section in the medical volume data at that time and the ultrasound scanning plane (that is, the ultrasound probe 18a). That is, the position and posture information conversion unit 66 can convert position and posture information indicating the position and the posture of the ultrasound probe 18a into converted position and posture information in the model coordinate system.

The scanned region specifying unit 68 specifies a scanned region in the three-dimensional model 56 representing the target tissue T, which is a region that has been scanned with an ultrasound beam (that is, where a received signal has been acquired). The processing of the scanned region specifying unit 68 will be described in detail below with reference to FIGS. 7 to 10.

FIG. 7 is a diagram showing a first example of a model scanning plane MCS in the model coordinate system. FIG. 7 shows the position and the posture of the ultrasound probe 18a in the model coordinate system (that is, the position and the posture indicated by the converted position and posture information). As described above, the ultrasound scanning plane is determined based on information known to the ultrasound diagnostic apparatus 18, such as the scan width and the penetration depth, and the position and the posture of the ultrasound probe 18a. Therefore, the scanned region specifying unit 68 specifies the model scanning plane MCS, which is the ultrasound scanning plane in the model coordinate system, based on the known information and the converted position and posture information. In the present specification, in order to distinguish between an ultrasound scanning plane in real space where scanning with an ultrasound beam actually is performed and a virtual ultrasound scanning plane specified in a model coordinate system, the ultrasound scanning plane in real space will be referred to as an ultrasound scanning plane as it is, and the virtual ultrasound scanning plane in the model coordinate system will be referred to as the model scanning plane MCS.

In a case in which an ultrasound beam is scanned on an ultrasound scanning plane corresponding to the model scanning plane MCS shown in FIG. 7 (in other words, in a case in which a transmission signal is supplied from the transmission/reception unit 40 to the ultrasound probe 18a), the scanned region specifying unit 68 specifies, as the scanned region, the region of the three-dimensional model 56 through which the specified model scanning plane MCS passes. In other words, the scanned region specifying unit 68 specifies a cross section of the three-dimensional model 56 defined by the model scanning plane MCS as the scanned region.

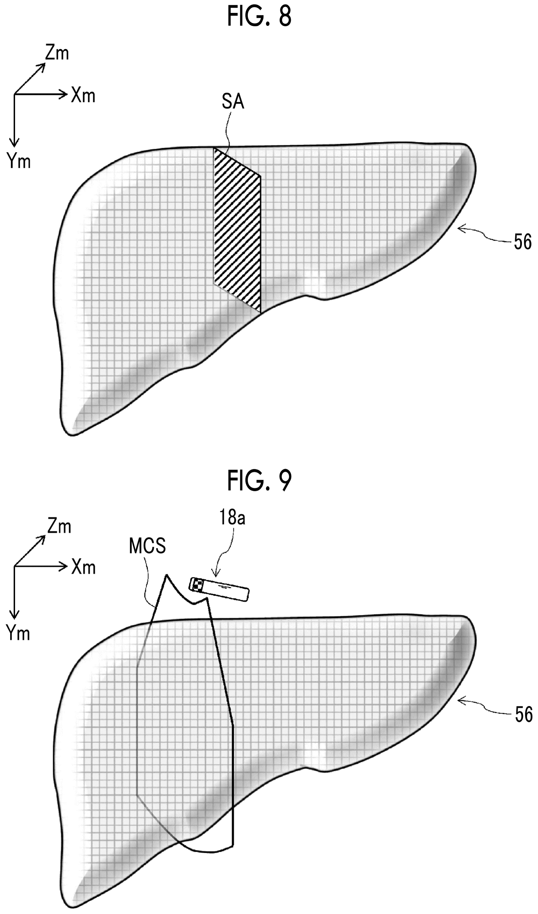

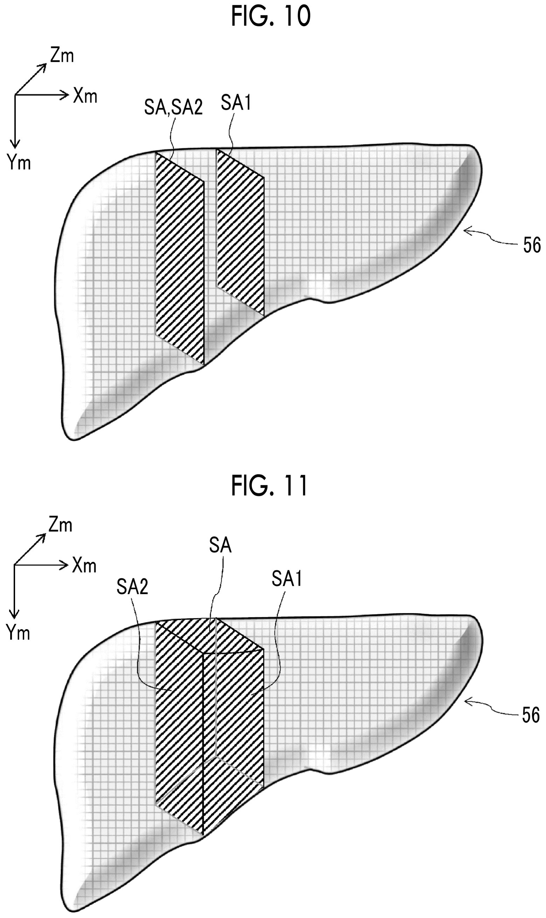

FIG. 8 is a diagram showing a first example of a scanned region SA. In a case in which the scanned region specifying unit 68 specifies a model scanning plane MCS as shown in FIG. 7, the scanned region specifying unit 68 specifies the region of the three-dimensional model 56 through which the model scanning plane MCS passes as the scanned region SA. In FIG. 8 (and FIGS. 10 and 11), the scanned region SA is indicated by diagonal lines. In the example of FIG. 8, the scanned region SA is a cross section of the three-dimensional model 56 cut by the model scanning plane MCS. Since the model scanning plane MCS shown in FIG. 7 has a scan width and a penetration depth sufficient to cross the three-dimensional model 56, in the example of FIG. 8, the entire cross section of the three-dimensional model 56 cut by the model scanning plane MCS is the scanned region SA, but in a case in which the model scanning plane MCS is too small to cross the three-dimensional model 56 (the scan width is small or the penetration depth is shallow), only a portion of the cross section of the three-dimensional model 56 cut by the model scanning plane MCS is specified as the scanned region SA. The scanned region specifying unit 68 stores information indicating the scanned region SA (the coordinate region of the scanned region SA) in the memory 54.

In a case in which the operator changes the position or the posture of the ultrasound probe 18a, the position or the posture of the ultrasound probe 18a in the model coordinate system changes, and the position and the posture of the model scanning plane MCS also change. FIG. 9 is a diagram showing a second example of the model scanning plane MCS in the model coordinate system. In a case in which an ultrasound beam is scanned on the ultrasound scanning plane corresponding to the model scanning plane MCS shown in FIG. 9, the scanned region specifying unit 68 again specifies, as the scanned region SA, the region of the three-dimensional model 56 through which the model scanning plane MCS passes.

FIG. 10 is a diagram showing a second example of the scanned region SA. In a case in which the scanned region specifying unit 68 specifies a model scanning plane MCS as shown in FIG. 9, the scanned region specifying unit 68 specifies the region of the three-dimensional model 56 through which the model scanning plane MCS passes as the scanned region SA (SA2 in FIG. 10). The scanned region specifying unit 68 also stores information indicating the scanned region SA2 in the memory 54.

FIG. 11 is a diagram showing a third example of the scanned region SA. In a case in which the ultrasound probe 18a gradually moves the ultrasound scanning plane while performing scanning with the ultrasound beam from the position shown in FIG. 7 to the position shown in FIG. 9, the entire region of the three-dimensional model 56 between the scanned region SA1 and the scanned region SA2 is specified as the scanned region SA, as shown in FIG. 11.

In this way, the scanned region specifying unit 68 can specify one or more surfaces (cross sections) in the three-dimensional model 56 that are independent (discontinuous) as the scanned region SA, or can specify the three-dimensional scanned region SA as a collection of such surfaces.

The received signal evaluation unit 70 evaluates the received signal obtained as the ultrasound probe 18a scans the ultrasound scanning plane corresponding to the model scanning plane MCS specified by the scanned region specifying unit 68 (that is, defining the scanned region SA) with the ultrasound beam. More specifically, the received signal evaluation unit 70 evaluates the received signal from the viewpoint of whether the ultrasound tomographic image is sufficiently visible in a case in which the ultrasound tomographic image is formed based on the received signal.

Various methods for evaluating the received signal are conceivable, but for example, a learning model for image analysis such as U-net can be used. For example, U-net is trained to receive an ultrasound tomographic image as an input and detect artifacts such as side shadows that appear in the ultrasound tomographic image. Therefore, the received signal evaluation unit 70 inputs the ultrasound tomographic image formed by the image forming unit 44 based on the received signal into the trained U-net, and detects artifacts and the like that occur in the ultrasound tomographic image based on the output of the U-net. The received signal evaluation unit 70 then evaluates the received signal corresponding to the ultrasound tomographic image in accordance with the quantity and the quality of the detected artifacts.

The display controller 46 causes the display 48 to display the three-dimensional model 56. In addition, the display controller 46 displays the scanned region SA specified by the scanned region specifying unit 68 and the unscanned region, which is a region that has not been scanned with the ultrasound beam, in the three-dimensional model 56 in different display modes.

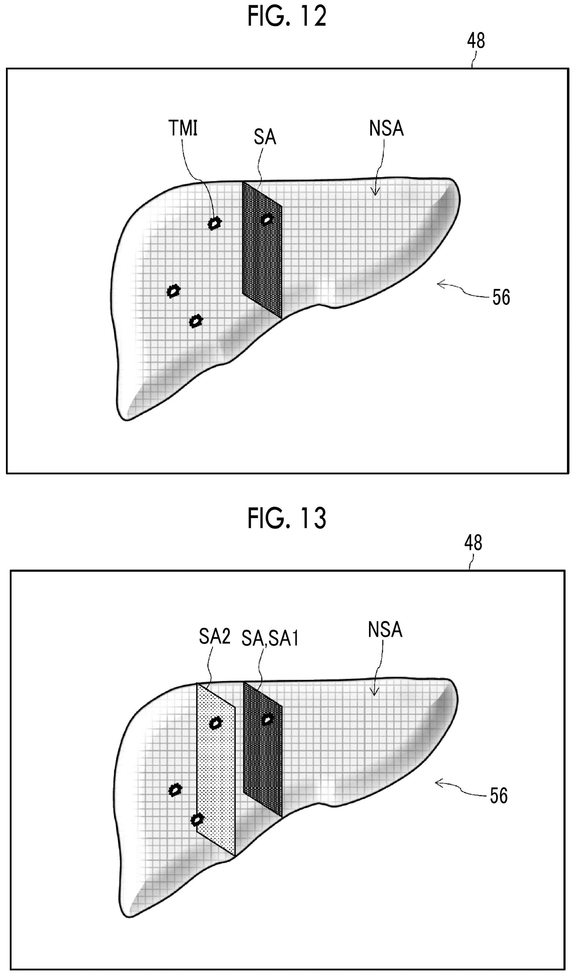

FIG. 12 is a diagram showing a first display example of the three-dimensional model 56. In the example of FIG. 12, the display controller 46 displays the three-dimensional model 56 by filling in the scanned region SA with a predetermined color (represented by shading in FIG. 12) and leaving the unscanned region NSA unfilled. In the example of FIG. 12, the scanned region SA is one surface, but in a case in which a three-dimensional region within the three-dimensional model 56 is specified as the scanned region SA as shown in FIG. 11, the display controller 46 displays the three-dimensional model 56 in which the three-dimensional scanned region SA is filled with a predetermined color.

The display controller 46 displays the scanned region SA and the unscanned region NSA in different display modes, so that the user can easily understand the region in the target tissue T that has been scanned with the ultrasound beam.

As described above, the three-dimensional model 56 has information indicating the coordinate region occupied by the portion of interest (the tumor TM in the present embodiment). Therefore, the display controller 46 may cause the display 48 to display information indicating the position of the portion of interest. In the present embodiment, as shown in FIG. 12, the display controller 46 displays the region occupied by the tumor TM on the three-dimensional model 56 by displaying a tumor image TMI in the coordinate region indicated by the tumor TM.

By displaying the scanned region SA and the unscanned region NSA in different display modes and displaying the tumor image TMI, the user can easily understand whether or not the tumor TM, which is the portion of interest, has been scanned with the ultrasound beam. In particular, in a case in which a plurality of tumors TM are scattered, as shown in FIG. 12, the display controller 46 displays a plurality of tumor images TMI, allowing the user to easily understand tumors TM that have not been scanned with the ultrasound beam.

FIG. 13 is a diagram showing a second display example of the three-dimensional model 56. The display controller 46 may display the scanned region SA such that the evaluation results of the received signal evaluation unit 70 for the received signals obtained from the ultrasound scanning plane CS corresponding to the model scanning plane MCS that defines the scanned region SA are known. For example, in a case of filling the scanned region SA with a predetermined color, the display controller 46 may display the scanned region SA in such a manner that the better the evaluation result of the received signal evaluation unit 70, the darker the color, and the worse the evaluation result, the lighter the color. For example, in the example of FIG. 13, the color of the scanned region SA2 is lighter than that of the scanned region SA1, which indicates that the evaluation result for the received signal corresponding to the scanned region SA2 is poor.

By displaying the scanned region SA such that the evaluation results of the received signal evaluation unit 70 are known, the user can easily understand regions that need to be scanned again with the ultrasound beam, even in a case in which the region has already been scanned with the ultrasound beam. For example, even though tumor TM has already been scanned with an ultrasound beam, in a case in which many artifacts occur in the ultrasound tomographic image formed from the received signals obtained thereby, the user may not be able to suitably understand the tumor TM. In this case, it is preferable to scan the tumor again with an ultrasound beam. In that case, the user can easily understand that the tumor needs to be scanned again with an ultrasound beam.

In the present embodiment, the display controller 46 displays the scanned region SA and the unscanned region NSA in different display modes by displaying the three-dimensional model 56 in which the scanned region SA is filled in, but the method of displaying the scanned region SA and the unscanned region NSA in different display modes is not limited thereto.

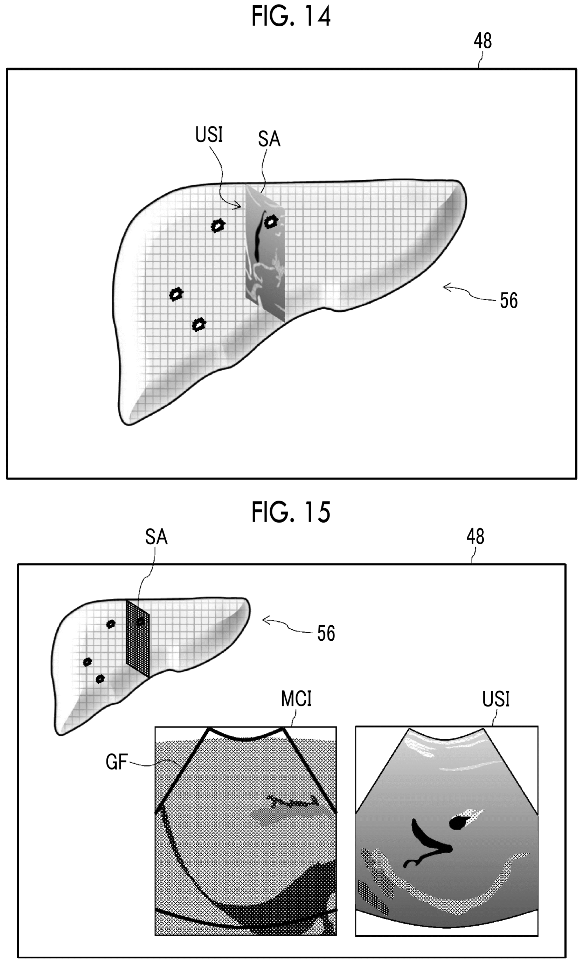

FIG. 14 is a diagram showing a third display example of the three-dimensional model 56. For example, as shown in FIG. 14, the display controller 46 may display the ultrasound tomographic image USI formed by the image forming unit 44 on a cross section as the scanned region SA defined by the model scanning plane MCS based on a received signal obtained by scanning an ultrasound scanning plane corresponding to the model scanning plane MCS with an ultrasound beam. In a case in which the three-dimensional scanned region SA, which is a collection of such cross sections, is specified, the display controller 46 may display the three-dimensional scanned region SA as ultrasound volume data.

FIG. 15 is a diagram showing a display example of the three-dimensional model 56, the medical tomographic image MCI, and the ultrasound tomographic image USI. The display controller 46 may cause the display 48 to display the three-dimensional model 56 showing the scanned region SA, the medical tomographic image MCI, and the ultrasound tomographic image USI. The medical tomographic image MCI is an image reconstructed by cutting out medical volume data received from the medical apparatus 12 or the medical image analysis server 14 at a cross section based on the converted position and posture information of the ultrasound probe 18a. In addition, the ultrasound tomographic image USI is an ultrasound tomographic image (that is, a real-time ultrasound tomographic image) formed based on received signals acquired at the current position and posture of the ultrasound probe 18a.

In a case in which the position and posture information of the ultrasound probe 18a is converted into the converted position and posture information, the image forming unit 44 can specify a cross section of the medical volume data that represents the same cross section as the current position and posture of the ultrasound probe 18a (that is, the current ultrasonic wave transmitting and receiving surface). Then, the image forming unit 44 can form the medical tomographic image MCI of the specified cross section. Similarly, the image forming unit 44 can form a (real-time) ultrasound tomographic image USI corresponding to the current ultrasonic wave transmitting and receiving surface. The display controller 46 can cause on the display 48 to display the medical tomographic image MCI and the ultrasound tomographic image USI formed in this way. That is, once the position and posture information of the ultrasound probe 18a is converted into the converted position and posture information, it is possible to always display the medical tomographic image MCI representing the current ultrasound scanning plane and the ultrasound tomographic image USI in a synchronized manner. That is, in a case in which the ultrasound scanning plane changes, both the medical tomographic image MCI and the ultrasound tomographic image USI represent the ultrasound scanning plane after the change. Both images always represent the same cross section.

By displaying the three-dimensional model 56 showing the scanned region SA, the medical tomographic image MCI, and the ultrasound tomographic image USI on the display 48, the operator can operate the ultrasound probe 18a such that the desired region in the three-dimensional model 56 is the scanned region SA while checking the medical tomographic image MCI or the ultrasound tomographic image USI corresponding to the current ultrasound scanning plane.

The display controller 46 may also display a guide image GF indicating the range of the ultrasound scanning plane in a superimposed manner on the medical tomographic image MCI. The medical tomographic image MCI is a rectangular image because it is configured by reconstructing medical volume data. On the other hand, in a case in which the ultrasound probe 18a performs sector scanning, the ultrasound beam is operated in a fan shape, and therefore the ultrasound scanning plane (that is, the region showing the state of the target tissue T in the ultrasound tomographic image USI) has a shape in which the lateral width increases as the depth increases. The range of the ultrasound scanning plane in the medical tomographic image MCI indicates the range in which scanning with the ultrasound beam can be performed at the position and the posture of the ultrasound probe 18a corresponding to the cross section of the medical tomographic image MCI.

By displaying the guide image GF in a superimposed manner on the medical tomographic image MCI, the user can easily understand which range in the medical tomographic image MCI is the scanned region SA.

Although the ultrasound diagnosis support apparatus according to the present disclosure has been described above, the ultrasound diagnosis support apparatus according to the present disclosure is not limited to the above-described embodiment, and various changes can be made without departing from the gist of the present disclosure.

For example, in the present embodiment, the ultrasound probe 18a is a drop-in type probe, but the ultrasound probe 18a may be another type of probe. For example, the ultrasound probe 18a may be a probe that is brought into contact with the body surface of the subject.

Furthermore, in each of the above embodiments, the ultrasound diagnostic apparatus 18 has the functions of the image forming unit 44, the display controller 46, the three-dimensional model forming unit 62, the probe position and posture detection unit 64, the position and posture information conversion unit 66, the scanned region specifying unit 68, and the received signal evaluation unit 70. However, these functions may not necessarily be exhibited by the ultrasound diagnostic apparatus 18. For example, these functions may be exhibited by other devices such as the medical image analysis server 14. Furthermore, all of the above-described functions may not be exhibited by one device, and the above-described functions may be exhibited by cooperation of a plurality of devices.

In the present embodiment, each process is executed by any computer. Further, any computer may execute these processes using a processor as hardware, a program as software, or a combination thereof. In this case, the processor is configured to cooperate with the program to execute various processes in the present embodiment, and can function as each unit or each means in the present embodiment. Further, the order in which the processes are executed by the processor is not limited to the order described above and may be changed as appropriate. Any computer may be a general purpose computer, a special purpose computer, a workstation, or other system capable of executing each process.

The processor may be configured with one or more pieces of hardware, and the type of hardware is not limited. For example, the processor may be configured with hardware such as a central processing unit (CPU), a micro processing unit (MPU), a programmable logic device such as a field-programmable gate array (FPGA), a dedicated circuit for executing specific processing such as an application-specific integrated circuit (ASIC), a graphics processing unit (GPU), or a neural processing unit (NPU). Additionally, the types of hardware may be a combination of different types of hardware. In a case in which a plurality of pieces of hardware are configured to execute one or more processes of a certain processor, the plurality of pieces of hardware may be present in devices physically separate from each other, or may be present in the same device. In addition, in any of the embodiments, the order of the processes performed by the processor is not limited to the order described above, and may be changed as appropriate. The hardware is configured by electrical circuits (circuitry) combining circuit elements such as semiconductor elements.

Further, the program may be software such as firmware or a microcode. The program may also be, for example, a group of program modules, each function of which may be implemented by a processor configured to execute each function. The program may be a program code or a plurality of code segments stored in one or more non-transitory computer-readable media (for example, storage media or other storages). The program may be stored in a plurality of non-transitory computer-readable media that are present in apparatuses physically separate from each other in a divided manner. A program code or a code segment may represent a procedure, a function, a subprogram, a routine, a subroutine, a module, a software package, a class, or any combination of instructions, data structures, or program statements. A program code or a code segment may be connected to another code segment or a hardware circuit by transmitting and receiving information, data, arguments, parameters, or memory content.

The present invention can also be applied to a program and a program product.

### Explanation of References

10: ultrasound diagnosis support system
12: medical apparatus
14: medical image analysis server
16: camera
18: ultrasound diagnostic apparatus
18a: ultrasound probe
30: probe detection mark
32: object detection mark
40: transmission/reception unit
42: signal processing unit
44: image forming unit
46: display controller
48: display
50: communication interface
52: input interface
54: memory
56: three-dimensional model
60: processor
62: three-dimensional model forming unit
64: probe position and posture detection unit
66: position and posture information conversion unit
68: scanned region specifying unit
70: received signal evaluation unit

## Claims

1. An ultrasound diagnosis support apparatus comprising:
a processor,
wherein the processor is configured to:
acquire a three-dimensional model of a target tissue formed based on medical volume data acquired by a medical apparatus other than an ultrasound diagnostic apparatus, each position of the three-dimensional model being represented by a coordinate in a model coordinate system;
acquire position and posture information indicating a position and a posture of an ultrasound probe that scans an ultrasound scanning plane including the target tissue with an ultrasonic wave;
convert the position and posture information into converted position and posture information in the model coordinate system;
specify a model scanning plane that is a virtual ultrasound scanning plane in the model coordinate system based on the converted position and posture information in a case in which the target tissue is scanned with an ultrasound beam, and specify a region of the three-dimensional model through which the model scanning plane passes as a scanned region; and
display the three-dimensional model on a display unit, and display the scanned region and an unscanned region, which is a region that has not been scanned with the ultrasound beam, in the three-dimensional model in different display modes.

2. The ultrasound diagnosis support apparatus according to claim 1,
wherein information indicating a position of a portion of interest in the target tissue is added to the three-dimensional model, and
the processor is configured to display the information indicating the position of the portion of interest on the display unit.

3. The ultrasound diagnosis support apparatus according to claim 1 or 2,
wherein the processor is configured to:
evaluate a received signal obtained by scanning an ultrasound scanning plane corresponding to the model scanning plane that defines the scanned region with the ultrasound beam; and
display the scanned region in a manner in which an evaluation result of the received signal is known.

4. The ultrasound diagnosis support apparatus according to any one of claims 1 to 3,
wherein the processor is configured to display, on a cross section as the scanned region defined by the model scanning plane, an ultrasound tomographic image formed based on a received signal obtained by scanning an ultrasound scanning plane corresponding to the model scanning plane with the ultrasound beam.

5. The ultrasound diagnosis support apparatus according to any one of claims 1 to 4,
wherein the processor is configured to:
display, on the display unit, a medical tomographic image reconstructed by cutting out the medical volume data at a cross section based on the converted position and posture information, and an ultrasound tomographic image formed based on a received signal acquired at a current position and posture of the ultrasound probe; and
display a guide image indicating a range of the ultrasound scanning plane in a superimposed manner on the medical tomographic image.

6. The ultrasound diagnosis support apparatus according to any one of claims 1 to 5,
wherein the processor is configured to detect a position and a posture of the ultrasound probe relative to a position and a posture of the target tissue based on a captured image obtained by imaging detection marks attached to the ultrasound probe and the target tissue with a camera.

7. An ultrasound diagnosis support program causing a computer to:
acquire a three-dimensional model of a target tissue formed based on medical volume data acquired by a medical apparatus other than an ultrasound diagnostic apparatus, each position of the three-dimensional model being represented by a coordinate in a model coordinate system;
acquire position and posture information indicating a position and a posture of an ultrasound probe that scans an ultrasound scanning plane including the target tissue with an ultrasonic wave;
convert the position and posture information into converted position and posture information in the model coordinate system;
specify an ultrasound scanning plane in the model coordinate system based on the converted position and posture information in a case in which the target tissue is scanned with an ultrasound beam, and specify a region of the three-dimensional model through which the ultrasound scanning plane passes as a scanned region; and
display the three-dimensional model on a display unit, and display the scanned region and an unscanned region, which is a region that has not been scanned with the ultrasound beam, in the three-dimensional model in different display modes.
